# EUROPEAN PATENT SPECIFICATION

(11) **EP 4 559 385 B1**
(45) Date of publication and mention of the grant of the patent: **21.10.2026**
(21) Application number: 24214375.8
(22) Date of filing: 21.11.2024
(51) Int. Cl.: A61B 5/0531, A61B 5/0295, A61B 5/0535

(54) **DEVICE AND METHOD FOR MEASUREMENT OF ELECTRICAL BIOIMPEDANCE**
VORRICHTUNG UND VERFAHREN ZUR MESSUNG DER ELEKTRISCHEN BIOIMPEDANZ
DISPOSITIF ET PROCÉDÉ DE MESURE DE BIOIMPÉDANCE ÉLECTRIQUE

(30) Priority: 24.11.2023 EE 202300029
(43) Date of publication of application: 28.05.2025
(73) Proprietor: Estvital Technologies OÜ, 10115 Tallinn Harju maakond (EE)
(72) Inventor: Min, Mart, 13424 Tallinn (EE); Rist, Marek, 76505 Saue (EE)
(74) Representative: Koitel, Raivo

(56) References cited:
- US-A1- 2015 216 441
- US-A1- 2018 078 148
- US-A1- 2019 285 717
- US-B2- 10 952 633
- OJARAND JAAN ET AL: "Derivation of Bioimpedance Model Data Utilizing a Compact Analyzer and Two Capacitive Electrodes: A Forearm Example", IEEE TRANSACTIONS ON BIOMEDICAL CIRCUITS AND SYSTEMS, IEEE, US, vol. 16, no. 5, 13 September 2022 (2022-09-13), pages 891 - 901, XP011928477, ISSN: 1932-4545, [retrieved on 20220914], DOI: 10.1109/TBCAS.2022.3206666
- ANAND GAUTAM ET AL: "Bioimpedance analysis as a tool for hemodynamic monitoring: overview, methods and challenges", vol. 42, no. 3, 6 April 2021 (2021-04-06), GB, pages 1 - 31, XP093233598, ISSN: 0967-3334, Retrieved from the Internet <URL:https://iopscience.iop.org/article/10.1088/1361-6579/abe80e/pdf> DOI: 10.1088/1361-6579/abe80e
- YU YANG ET AL: "The Investigation of Bio-impedance Analysis at a Wrist Phantom with Two Pulsatile Arteries", vol. 14, no. 6, 1 January 2023 (2023-01-01), Cham, pages 810 - 826, XP093233599, ISSN: 1869-408X, Retrieved from the Internet <URL:https://link.springer.com/article/10.1007/s13239-023-00689-9/fulltext.html> DOI: 10.1007/s13239-023-00689-9

## Description

### Field of technology

The invention belongs to the field of medical technology. More specifically, the invention relates to body-worn medical devices and their use for assessing of a patient's health status.

### Prior art

A multi-functional wrist-worn blood pressure monitoring device (US 2017/0340209, Apple Inc, published November 30, 2017) is known, which has various sensors configured to non-invasively engage with the skin on the user's wrist to sense various physiological signals from the user's cardiovascular system. The various sensors comprise a pulse transit time (PTT) sensor, an ECG sensor, an electrical bioimpedance (EBI) sensor. The device uses a classic 4-electrode impedance measurement system for measuring the electrical bioimpedance of the body from the skin surface. The device comprises an excitation current generator, the first and second excitation current electrodes, the first and second voltage pickup electrodes and a voltage meter. The voltage drop caused by the current passing through the body impedance is picked up by the first voltage electrode and the second voltage electrode through the corresponding contact impedances of the first electrode and the second electrode. The disadvantage of the solution is that the contact impedances in the path of the current electrodes remain outside the measured body impedance according to the solution principle, but actually it remains undetermined which body area the excitation current passes through and therefore it remains undetermined which region of the body impedance we actually measure. To be noted that the contact resistances of the voltage electrodes with the measured body also causes uncertainty. The problem can be solved by using a voltage meter with a symmetrical differential input, but this is a complex and expensive solution. In addition, a measurement error inevitably occurs due to the presence of a common mode voltage on both the voltage electrodes, suppression of which is not perfect. Known method and apparatus for measuring bioimpedance (US 10952633, Samsung Electronics, published March 23, 2023) uses a 4-electrode measurement circuit and comprises a first electrical signal generator configured to generate a first electrical signal to measure the bioimpedance of an object. The apparatus also includes a second, the compensation signal generator configured to generate a compensation signal to compensate the bio-signal measured with the help of the first electrical signal generator, and an amplifier configured to amplify the compensated bio-signal. In addition to the contact impedances of the four measurement electrodes, body impedances not included in the measurement chain are shown as potential sources of measurement errors. To eliminate the influence of the contact impedances of the electrodes of unknown size and the unknown body impedance components, the second generator with additional electrodes has been introduced, which generates an electrical compensation signal for measuring the reference impedance of a body part similar to the object, the corresponding voltage of which is subtracted from the voltage signal on the voltage electrodes. This compensates the influence of the contact impedances of the electrodes and the unknown body impedances on the measurement result. The disadvantage of the presented solution is the addition of electrodes and electronic components with additional electrical connections between them. The disadvantage is the increase in the complexity of the device and the fact that the efficiency of the compensation depends on poorly defined circumstances violating the success of the compensation effect.

Known are the methods for using wearable devices to monitor a patient's health status by measuring the electrical bioimpedance of their cardiovascular and pulmonary systems (Anand, G., et al 2021, Topical Review: Bioimpedance analysis as a tool for hemodynamic monitoring: overview, methods, and challenges. Physiol. Meas. DOI: 10.1088/1361-6579/abe80e), especially regarding measuring the impedance of the patient's wrist arteries. (Yu, Y., et al. The Investigation of Bioimpedance Analysis at a Wrist Phantom with Two Pulsatile Arteries. Cardiovasc. Eng. Technol., October 17, 2023. DOI: 10.1007/s13239-023-00689-9).

The closest analogue to the presented solution in terms of technical nature is a bioimpedance-based pulse waveform sensing system and method (US 2018/0078148, March 22, 2018. Cohn, G. A., et al. Bioimpedance based pulse waveform sensing, Microsoft Technology Licensing, published March 22, 2018), where the system comprises a pressure measuring sensor and an arterial pulse wave sensor based on bioimpedance measurement. The arterial pulse wave is determined by measuring the voltage difference between two voltage electrodes. The current generator in the bioimpedance measuring device generates an excitation current to the object through the current electrodes, and the measuring device detects and outputs the voltage difference created by the excitation current in the tissues of the wrist area, which is proportional to the bioimpedance between the voltage electrodes. A four-electrode circuit is used to measure the bioimpedance, where four short strip electrodes placed on the object - radial artery - perpendicularly. The disadvantage of this solution is that the electrical resistances between the biological object (artery) and the electrodes are on average about 100 times greater than the measured electrical bioimpedance Z and more than 1000 times greater than the average informative change in electrical bioimpedance. Measuring such a small impedance change against the background of large and only limitedly accurately compensated extraneous impedance components is difficult. Another disadvantage is the use of short strip electrodes placed perpendicularly to the measurement object, such as an artery, aorta, or other blood vessel, which, compared to the known point and round electrodes (US 10952633, Samsung Electronics, published March 23, 2021), does provide an opportunity to somewhat increase the sensitivity, but it is far from decisive improvement, because the voltage response between the two voltage electrodes remains very small when a pulse wave arrives in comparison with unstable contact impedances between the electrodes and the biological object. The reliability of the information obtained by measurement suffers significantly.

The primary and greatest disadvantage of the closest analogue solution is that the measured impedance Z = 10-50 Ω of a biological object is on average 100 times smaller than the contact resistance between the excitation current electrode and the body in the order of r1 ≈ r2 = 1000-5000 Ω, while the informative change of the measured impedance ΔZ = 1-5 Ω is only one thousandth of it. Measuring such small impedance changes against the background of large and still unstable contact resistances is very problematic.

Another major drawback concerns electrodes with configuration in which the four point electrodes or four short strip electrodes (two excitation current electrodes and two voltage pickup electrodes) are placed perpendicularly to the biological object, which do not allow to determine the trajectory of the excitation current *i* between the excitation electrodes with sufficient accuracy. This is a reason the impedance measurement result Z = V / *i is* not accurate neither stable, since it is not clear what part of the excitation current passes through the measured impedance Z and gives the measurement result in the form of a voltage drop V.

Known is US 2015/216441 A1 (Khalfallah Kamel, et al, published August 6, 2015) which discloses an electrophysiological analysis system and a device.

The closest method for measurement of the electrical bioimpedance is the procedure described in the US patent application (2018/0078148, March 22, 2018. Cohn, G. A., et al. Bioimpedance based pulse waveform sensing, Microsoft Technology Licensing, March 22, 2018), which deals with the recording of a blood pulse curve together with finding of parameters of this curve, such as the value of blood pressure and the characteristics of its change in real time from the results of electrical bioimpedance measurement of the wrist. The method is distinguished by the fact that instead of the usual four-point electrodes (2 electrodes for introducing the excitation current and 2 electrodes for voltage collection), short strip electrodes are used, which are placed on the wrist in a position perpendicular to both the radial and ulnar arteries. The perpendicular position of the electrodes allows to somewhat improve the signal/noise ratio compared to using of point electrodes, however, the level of the pulse signal remains weak, because the size of the non-informative and unstable contact resistances (r1, r2) between the electrode and the biological object exceeds the size of the measured bioimpedance Z many times and is up to thousands of times greater than the value of the informative variation ΔZ of the bioimpedance Z. This significantly limits the reliability and usability of the measurement method described above in clinical applications. An attempt has been made to solve the problem by improving the method (US 10952633, Samsung Electronics, published March 23, 2021), which consists in adding separate specific electrodes for measuring the resistances between the electrode and the body and then subtracting the obtained result from the original measurement results. This technique improves the accuracy of the initial measurement results, but does not lead to a decisive improvement, because the additional measurement performed with similar, but still different electrodes, and moreover, the bioimpedance of the same biological body measured, but from a different location, however. Therefore, subtracting the measurement results obtained using additional electrodes is not a sufficiently effective method for eliminating uncertainty.

The measurement method related to the closest technical solution has the following disadvantages.

The first disadvantage of the known measurement method is that it is not possible with the known electrodes to direct the entire generated excitation current *i* exactly through the region the impedance Z of which we want to measure. The path of the excitation current *i* affected by the large and poorly known contact resistances r1 and r2, through which the current electrodes are connected to the body part. As a result of the measurement, we do not know exactly what impedance region of the biological object we are measuring.

Another disadvantage of the known measurement method is the generation of a constant value excitation current throughout the measurement frequency range, while the safety standards allow the higher excitation currents at higher frequencies. Therefore, the power of excitation current cannot used to its maximum extent to achieve the best signal-to-noise ratio and measurement accuracy, especially at higher frequencies above 10 kHz.

### Summary of the invention

The aim of the invention is to develop a device and method for accurate measurement of electrical bioimpedance to increase the reliability of the results. Known electrical bioimpedance measurement devices comprise a measuring device, excitation current electrodes and voltage pickup electrodes, electrical contact resistances (r1, r2) between the excitation current electrodes and the biological object, and a generator.

Known devices have disadvantages that limit their scope of application, especially in the field of medical diagnostics. To eliminate the disadvantages, the invention comprises the following improvements.

The following components with their interconnections have been additionally introduced into the electrical bioimpedance measurement device: an active electrode system, containing an operational amplifier and a current-impeding circuit, and an analog interface with inputs connected the outputs of three voltage signals - one of these is originating from the generator and two others (V1 and V2) from the active electrode system - which carry information about the bioimpedance Z of the biological object and its variation ΔZ. In the input of the analog interface there is a summator, which includes a first summing unit, a second summing unit and an amplifier, which separate the signal components corresponding to the impedance Z and its variation ΔZ. The summator is followed by a demodulator located in the analog interface, wherein there are two multipliers and a reference signal formator, and a low pass filter and a band pass filter, in which a signal carrying information about the impedance Z and a signal carrying its variation ΔZ, are demodulated separately but both synchronously with the generator voltage. One of the four voltage inputs of the measuring device is connected to the output of the operational amplifier for measuring the voltage V3 emanating from the excitation current electrode, and the other voltage input is connected to the voltage pickup electrode for measuring the voltage V1 emanating from it, whereas the remaining two voltage inputs of the measuring device are connected to the two outputs of the analog interface for measuring the demodulated output voltages DZ and DΔZ, which reflect the impedance Z and its variation ΔZ.

**Supplementing** the known devices with inventive elements enables to eliminate the following disadvantages.
1. Relatively high, unstable, and poorly defined contact resistances (r1, r2) between the excitation current electrodes and the biological object cause disturbing uncertainties up to the point of invalidating the measurement results.

This disadvantage is eliminated by introducing an active electrode system, which contains an inverting operational amplifier so that the excitation current electrodes are arranged in series through the electrode-body contact resistances r1 and r2 and the biological object in the negative feedback loop of the operational amplifier situating between the output and inverting input of the operational amplifier, which reduces the effect of the resistances r1 and r2 on the measurement result by a factor close to the gain of the operational amplifier without feedback. Since the average value of the gain factor of the operational amplifier is 100,000, the effective influence of the contact resistances r1 and r2 is only tens of milliohms (MΩ) from the informative variations within the range of ΔZ = 1 - 5 Ω. The misinformation effect of the contact resistances r1 and r2 becomes negligible.

2. The use of a constant excitation current *i* mostly used in the entire measurement frequency range does not enable the use of the permissible increase in the excitation current in living organisms at higher frequencies, because of which the possibility for increasing the measurement accuracy in higher operating frequency range remains unrealized.

This shortage eliminated by introducing a frequency dependent current-impeding circuit, switched between the generator's output and the inverting input of the operational amplifier, the frequency characteristics of the resistance R of which are as similar as possible to those of the impedance Z of the measured biological object. This enables us to achieve the highest permissible excitation current *i* value at all frequencies, but especially at high frequencies (f ≥ 10 kHz). We obtain the best signal/noise ratio at all operating frequencies, which makes it possible to measure with the least measurement error in the entire measurement frequency range.

3. Point electrodes or short strip electrodes placed perpendicular to the biological object in the known devices do not allow us to determine the trajectory of the excitation current *i* between the excitation current electrodes with sufficient accuracy. The current dissipates in the biological object, which results in excitation current leakage. Due to the undetermined leakage current, the result of the impedance Z = V / *i* measurement is also not accurate or stable, since the size of the part measuring the excitation current is not exactly known. We obtain an inaccurate measurement result in the form of the voltage drop VZ.

In the invention, the above-mentioned drawback is eliminated by using long strip electrodes, constituting an electrode system surrounding the biological object containing the measured bioimpedance Z in such a way that on one side of it there are placed one excitation current electrode and one voltage pickup electrode constituting one pair of electrodes, and on the other side there is another voltage pickup electrode and another excitation current electrode constitutes another pair of electrodes, whereby the voltage pickup electrodes have typically a wider grip than the dissipation of excitation current. There are specific electrode configurations, selection of which depends on the certain shape of the biological object, surrounded with electrodes to measure the bioimpedance Z in both a straight line and curved manner, capturing it in either an open or closed form. As a result, we achieve the minimal current leakage, which enables the entire generated excitation current *i* to pass through the measured bioimpedance Z, ensuring thereby the best measurement accuracy, especially for the informative variation ΔZ of biological object's bioimpedance Z.

4. A very troublesome drawback of the known solutions is the problematics connected with measuring the informative variation ΔZ constituting only a few hundredths or even thousandths of the total impedance Z. Uncertainties can cause large measurement errors in the presence of disturbances and noise, the measurement results may even prove to be unsuitable due to an excessively large measurement errors (error ≥30%).

To eliminate the drawback, a summator introduced into the input of the analog interface. It comprises the first and second summing units separating the voltage components VZ and VΔZ corresponding to the impedance Z and its variation ΔZ. The demodulator following the summator, detects synchronously with the generator voltage separately the voltage VZ corresponding to the bioimpedance Z and the amplified voltage signal VΔZ carrying information about its variation thereby. As a result, the accuracy and informativeness of the measurement results, especially of variation ΔZ improved.

5. The deficiency of the known solutions manifested in the fact that the value of the contact resistances r1 and r2 exceeds the value of the measured bioimpedance Z. Because of that, the measurement can lead to a nonlinear operation of the active electrode system and cause overloading. As a result, the errors can distort the measurement results completely up to the point of being fully unsuitable.

In the invention, the problem solved by means of control measurements. The first voltage pickup electrode connected to a measuring device, the voltage V1 measured there must remain below the level V1 ≤ permissible to avoid fatal measurement errors. Secondly, the excitation current electrode located at the output of the operational amplifier connected directly to the measuring device, the measurement result V3 of which must remain below the level V3 ≤ permissible to avoid nonlinear distortions of signals. If the given condition are not met, the correctness of attachment the electrode onto the body is to be checked, or the too high magnitude of the excitation current must be reduced. This way the formation of nonlinear distortions is avoided, and the large measurement errors are prevented.

The method of measuring electrical bioimpedance comprises the following steps:
- placing the excitation current electrodes and voltage pickup electrodes on a biological object, generating, and directing the electrical excitation current through the biological object via the excitation current electrodes;
- modulating the voltage generated in the biological object by the excitation current directed through the electrical bioimpedance Z of the biological object and recording the voltage modulated by the electrical bioimpedance Z on the voltage pickup electrodes;
- demodulating and measuring the voltages taken from the voltage pickup electrodes and deriving the value of the electrical bioimpedance Z=V/i by dividing the voltage drop V measured on the voltage pickup electrodes by the excitation current *i* directed through the biological object.

### List of figures

Fig 1. The structure of the electrical bioimpedance measurement device and the connections between its main components - the active electrode system (AES), the analog interface (AI), the generator (G) and the measuring device (MD).
Fig 2. The active electrode system (AES) connected with the analog interface (AI) and the generator (G).
Fig 3. The electrical circuit diagram of the active electrode system (AES) connected with the generator (G).
Fig 4. The excitation current flow and voltage distribution between the electrodes.
Fig 5. The electric diagram of the current-impeding circuit (R) comprising the resistor r3.
Fig 6. The electric diagram of the current-impeding circuit (R) comprising the parallel resistor rp1 connected in parallel with the resistor r3 through the capacitance C1.
Fig 7. The electric diagram of the current-impeding circuit (R), which comprises a parallel resistor rp1 connected in parallel with the resistor r3 through the capacitance C1 and a parallel resistor rp2 connected through the capacitance C2.
Fig 8. Structural diagram of the analog interface (AI).
Fig 9. Structural diagram of the summator (Σ1) containing in the analog interface (Al).
Fig 10. Structural diagram of demodulator (DEM) containing in the analog interface (Al).
Fig 11. An active electrode system (AES), where open electrodes are in parallel to the curvature area of the object.
Fig 12. An active electrode system (AES), where open electrodes are located opposite each other in arc-shape form to the area of the object.
Fig 13. An active electrode system (AES), where electrodes form a sector of concentrical location.
Fig 14. An Active electrode system (AES) consisting of closed electrodes located concentrically on the object (the letter d represents the inner diameter of the electrode (5).
Fig 15. The circuit diagram of the active electrode system (AES) with concentric electrodes, where the outer excitation current electrode 6 is connected to the output of the operational amplifier 9 and the innermost excitation current electrode 5, which has been reduced to a surface electrode (d → 0), is connected to the inverting input of the operational amplifier 4.
Fig 16. The circuit diagram of the active electrode system (AES) with concentric electrodes, but different from a circle, where the innermost excitation current electrode 5 forms a line electrode that is connected to the inverting input of the operational amplifier.
Fig 17. Fragment of the excitation current electrode 6 and the voltage pickup electrode 8 running in parallel to the curvature area of the biological object (see Fig 11), which are formed from serially connected point electrodes.

### Embodiments of the invention

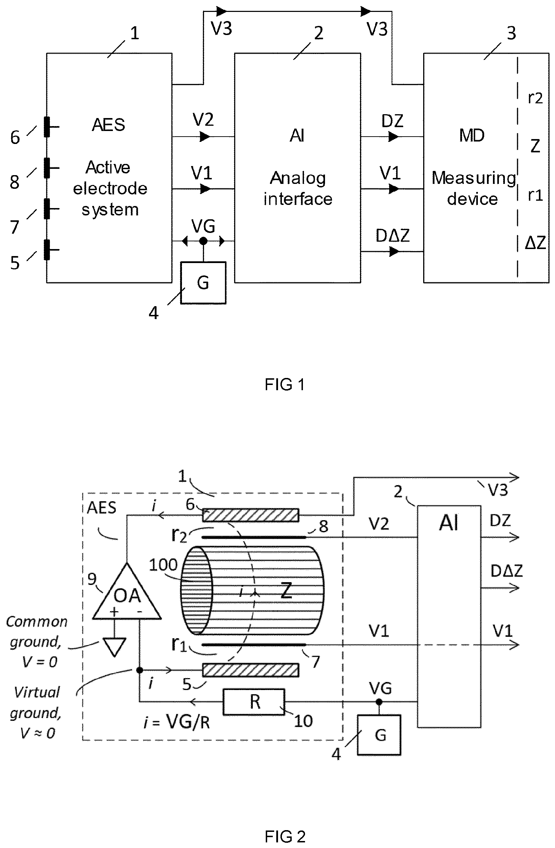

The device for measuring electrical bioimpedance in Fig 1 consists of an active electrode system (AES) 1, an analog interface (AI) 2, a measuring device (MD) 3 and a generator (G) 4, whereby the active electrode system (AES) 1 (Fig 1, Fig 2) comprises two excitation current electrodes (5, 6) and two voltage pickup electrodes (7, 8) and an operational amplifier (OA) 9, a current-impeding circuit (R) 10, contact resistances r1 and r2 and a measured biological object 100 with a bioimpedance Z.

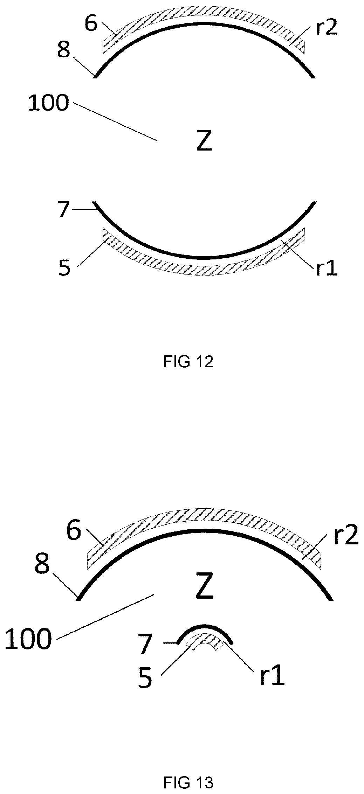

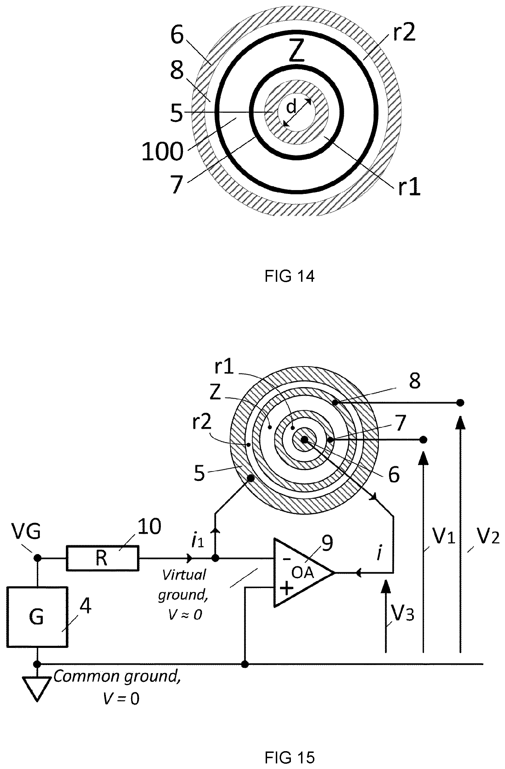

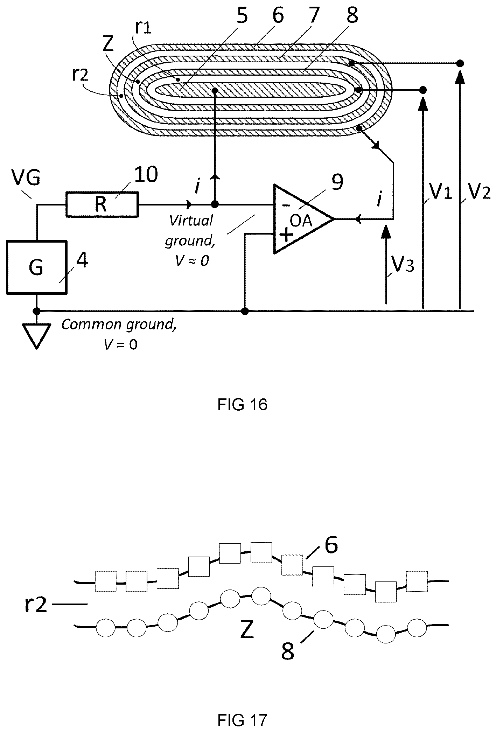

The electrodes 5, 6, 7, 8 (Fig 2) are grouped as a pair (5, 7) of one excitation current electrode 5 and one voltage pickup electrode 7, and as a pair (6,8) of another excitation current electrode 6 and another voltage pickup electrode 8, which on the biological object 100 are arranged as strip electrodes so that on one side of the measured bioimpedance Z there is one pair of strip electrodes (5, 7) and on the other side there is another pair of strip electrodes (6, 8), see Fig 2 and Figs 11 to 16, whereby the electrodes (5-8) may, if necessary, be formed as closed contours (Figs 14 to 16), which are designed to surround the measured impedance Z of the biological object 100. Fig 17 shows an example where the strip electrodes presented as a series of connected point electrodes.

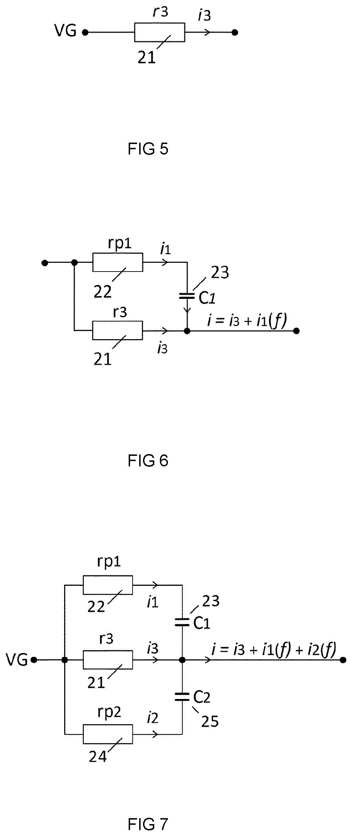

The simplest variant (Fig 5) of the current-impeding circuits 10 (Figs 5 to 7) comprises one resistor (r3) 21, the more complex basic version (Fig 6) comprises, in addition to the resistor (r3) 21, a parallel resistor (rp1) 22 and a capacitor (C1) 23, the most complex third version (Fig 7) comprises in addition to the resistor (r3) 21, the parallel resistor (rp1) 22 and the capacitor (C1) 23, also another parallel resistor (rp2) 24 and a second capacitor (C2) 25.

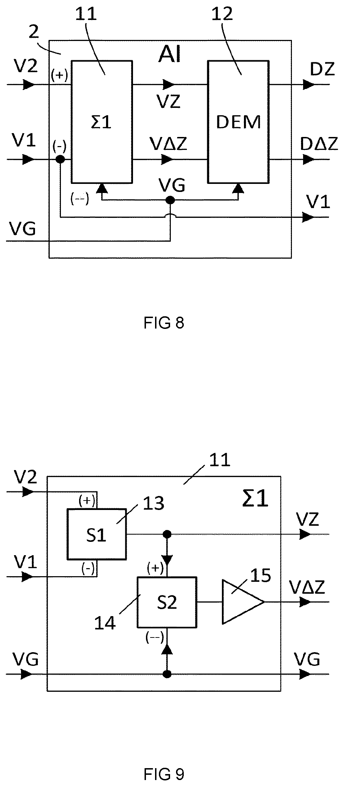

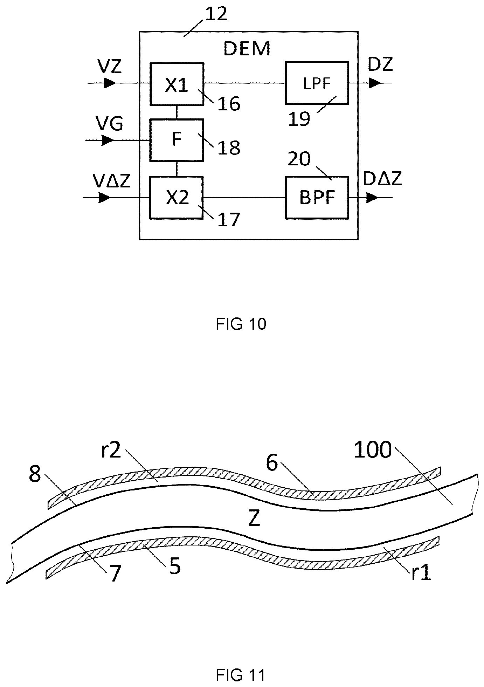

The analog interface (AI) 2 in the Fig 1, Fig 2 and Figs 8 to 10, comprises a summator (Σ1) 11 and a demodulator (DEM) 12 (Fig 8), wherein the summator (Σ1) 11 consists of a first summing unit (S1) 13 and a second summing unit (S2) 14, and an amplifier 15 (Fig 9), the demodulator (DEM) 12 consists of the first multiplier (X1) 16, the second multiplier (X2) 17, and the formator (F), low-pass filter (LPF) 19 and bandpass filter (BPF) 20 (Fig 10).

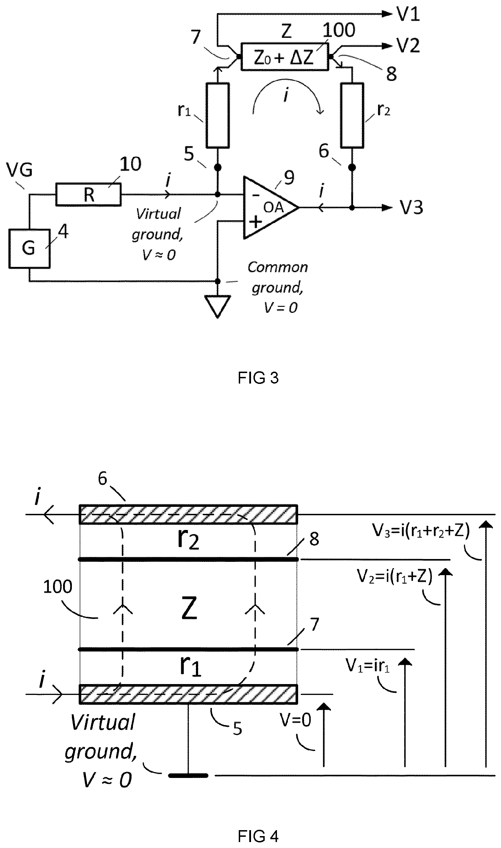

The active electrode system (AES) 1 (Fig 1, Fig 2) is connected to the biological object 100 through the contact resistances r1 and r2 of excitation current *i* electrodes (5, 6) of the bioimpedance Z to be measured, whereby one excitation current electrode 6 is connected to the output of operational amplifier 9 and the other excitation current electrode 5 is connected to the inverting input (-) of the same operational amplifier 9, the non-inverting input (+) of which is connected to the common ground of the device with potential V=0 (Fig 2, Fig 3). The voltages V1 and V2 taken from the measured impedance Z by the voltage pickup electrodes (7, 8), see Figs 2 to 4.

The current-impeding circuit 10 (Figs 1 to 3) is connected to the inverting input (-) of the operational amplifier (OA) 9, through which this inverting (-) input is connected to the output of the generator (G) 4, which is also connected to the input of the analog interface (AI) 2. The current-impeding circuit (R) 10 comprises a resistor r3 (Fig 5), to which, in a basic variant (Fig 6), the parallel resistor rp1 with serially switched capacitor C1 are connected in parallel, and, in the most complex case (Fig 7), another parallel resistor rp2 with serially switched capacitor C2 are connected parallelly.

The voltage pickup electrodes 7 and 8 are connected to the inputs (Fig 8) of the summator (Σ1) 11 located in the analog interface (AI) 2 in Fig 2, whereby the voltage pickup electrode 7 has a connection with its inverting input (-) with voltage V1 and the voltage pickup electrode 8 relates to its non-inverting input (+) with voltage V2 (Fig 8). The output of the generator 4 with voltage VG (Fig 1, Fig 2 and Fig 8) is connected to the second inverting input (--) of the summator 11. The output from the excitation current electrode 6 with a voltage V3 is connected directly to the input of the measuring device (MD) 3, see Fig 1 and Fig 2.

The inverting input (-) of the first summing unit (S1) 13 (Fig 9) situating in the summator (Σ1) 11 (Fig 8), represents also the inverting input (-) of the summator 11, which is connected to the voltage pickup electrode 7 with the voltage V1, and the non-inverting input (+) of the summing unit (S1) 13, being also the non-inverting input (+) of the summator 11, is connected to the voltage pickup electrode 8 with voltage V2 (Fig 2, Fig 4 and Fig 8). The output of the first summing unit (S1) 13 with voltage VZ is connected to the non-inverting input (+) of the second summing unit (S2) 14 as well as to one input of the demodulator (DEM) 12 (Fig 8), whereas the output of the generator 4 with voltage VG is connected to the inverting input (--) of the second summing unit (S2) 14. The output of the second summing unit (S2) 14 is connected to the input of the amplifier 15, the output of which with voltage VΔZ is connected to the second input of the demodulator DEM (12) located in the analog interface (AI) 2 (Fig 8), to the reference input of which the output of the generator 4 with voltage VG is connected. The two outputs of the demodulator (DEM) 12 with demodulated voltages DZ and DΔZ are connected to the two inputs of the measuring device (MD) 3, the output from the voltage pickup electrode 7 with voltage V1 is connected to the separate input of the measuring device (MD) 3 through the analog interface (AI) 2.

Operation of the device. The alternating excitation current *i* from the generator (G) 4 is directed through the current-impeding circuit (R) 10 to the inverting input (-) and through the negative feedback of the operational amplifier 9, while passing through the impedance Z of the biological object 100 and following through the contact resistances r1 and r2 of the excitation current electrodes 5 and 6 to the output of the operational amplifier 9 (Fig 2, Fig 3). The current-impeding circuit 10 imitates the measurable bioimpedance Z of the biological object 100 in terms of the properties and parameters of its resistance R. Due to the high gain of the operational amplifier 9, its inverting input (-) and the excitation current electrode 5 are connected to receive the virtual ground potential V ≈ 0 (Figs 2 to 4). Let us note that in different applications, especially in medical diagnostics, the most informative is the variation ΔZ ≈ (1-10) Ω of the full bioimpedance Z = Z0 + ΔZ (Fig 3), whereas the variation ΔZ consists only 1/1000 to 1/100 of the base value Z0 ≈ 1000 Ω.

The contact resistances r1 and r2 exceed the impedance change ΔZ value by hundreds of times, but the electrical scheme in Fig3 and the mathematical relationships presented in Fig 4 enable to calculate the values of the contact resistances r1 and r2 found from the voltages V1, V2, and V3 of the electrodes 6, 7 and 8 (Fig 2), and then eliminate their influence on the bioimpedance Z measurement results in the analog interface 2 (Fig 8, Fig 9), in which the summator (Σ1) 11 subtracts the voltages V2 and V1 of the voltage pickup electrodes 7 and 8 (Fig 9). As a result, we obtain the voltage VZ corresponding to the measured impedance Z. The subtraction operation is performed in the summing unit (S1) 13 of the summator (Σ1) 11. The variation ΔZ of the bioimpedance Z is obtained by the summing unit (S2) 14, where the voltage VG of the generator 4, proportional to the constant component Z₀ of the impedance Z, is subtracted from the voltage VZ. The tiny variation ΔZ obtained because of the subtracting operation, is amplified at the output of the summing unit (S2) 14 by the amplifier 15 giving the output signal in the form of the information-carrying voltage VΔZ. The voltages VZ and VΔZ are detected in the demodulator 12 by means of the first multiplier (X1) 16 and the second multiplier (X2) 17 synchronously with the voltage signal VG of the generator 4, which is converted into the reference signal in the shaping formator (F) 18 (Fig 10). The shaping consists in changing the waveform and shifting the phase for obtain the precise synchronization. The detected signal DZ, which corresponds to the impedance Z, is filtered out using low pass filter (LPF) 19 at the output of the first multiplier 16. The other detected signal DΔZ corresponding to the variation part ΔZ of the impedance Z, is filtered out by the bandpass filter (BPF) 20 from the output of the second multiplier 17.

The detected voltages DZ and DΔZ measured in the measuring device (MD) 3, represent the values of the bioimpedance Z and its variation ΔZ in digital form (Fig 1). The voltages V1 and V3, are also measured and evaluated in the measuring device (MD) 3 and the values of the contact resistances r1 and r2 are calculated and the fulfillment of the conditions for the correctness of the measurement process (r1 ≤ permissible) and (r2 ≤ permissible) are checked to avoid signal distortions causing measurement errors in the analogue interface 2.

Advantages of the invention in terms of the device
1. Introducing the inverting operational amplifier (OA) 9 for creating the active electrode system (AES) 1, makes possible to use a virtual ground with a potential very close to zero (V ≈ 0) for one of the excitation current electrodes 5, which is practically equal to the common ground potential of the device (V = 0), Figs 2 to 4. The structure of the device is simplified, energy consumption reduced, the reliability and measurement accuracy improved.
2. Measurement of the voltage V1 = i·r1 = (VG/ R)·r1 from the voltage pickup electrode 7 (Figs 1 to 4) at the excitation current *i* = VG/R, gives the contact resistance r1 = V1/*i* = R(VG/V1). Checking r1 ≤ permissible, ensuring the work of operational amplifier 9 in linear mode. Avoidance of nonlinear distortions ensures measurement accuracy.
3. The possibility of measuring the voltage V2= i(r1 +Z) of the voltage pickup electrode 8 (Figs 2 to 4) at the excitation current *i* = VG/R, gives the sum of resistances r1 + Z = R(V2/VG), from which the exact value for the impedance Z is acquired as Z = R[(V2-V1)/VG] regardless of the value of r1 if the requirement r1 ≤ permissible is followed.
4. A decisive reduction of the effect of contact resistance r2 is ensured by deep negative feedback from the output of the operational amplifier (OA) 9 through the contact resistance r2 of the excitation current electrode 6, the measured bioimpedance Z of the biological object 100, the contact resistance r1, and the excitation current electrode 5 to its inverting input (-), see Figs 2 to 4. In the case of a large amplification factor K ≥ 10⁵ of the operational amplifiers makes negligible the effect of the contact resistance r2 on the measurement accuracy, see Fig 2 and Fig 3.
5. The possibility of measuring the voltages V2 and V3 provides the value of the contact resistance r2 = (V3-V2)·(R/VG), which is necessary for the calculating and eliminating residual errors in the measured bioimpedance Z (Figs 2 to 4) for having smaller values of the gain factor K of the operational amplifier 9, for example, 10³ ≤ K ≤ 10⁵. Moreover, checking the condition r2 ≤ permissible ensures the work of operational amplifier 9 in the linear mode and avoids the errors from nonlinearities.
6. The use of the generator (G) 4 as a voltage source enables to generate the excitation current (*i* = VG/R) within a wide range of operating frequencies f by the aid of a simple electronic circuit. The use of current generators known from widespread practice of impedance measurements, requires complicated solutions that does not ensure distortion-free operation in a wide frequency range from f ≥ 100 kHz.
7. Using the current-impeding circuit 10 making the excitation current *i* dependable inversely to the frequency dependance of the measurable impedance Z, it means that the product (i x Z) is close to a constant in a wide range of measurement frequencies, for example, in the range f = 10 Hz to 10 MHz. This solution ensures the best measurement accuracy in conditions of noise and interference.
8. The measurement accuracy of the impedance Z and its variation ΔZ does not depend on the stability of voltage VG of the generator 4, because the same excitation current i = VG/R flows both through the impedance Z and the current-impeding circuit 10 with the resistance R. We obtain the dependence on the voltage ratio, Z = R(VZNG), but not on the magnitude of the excitation current i. We avoid the measurement errors due to instability of the voltage VG of the generator 4.
9. The long strip electrodes (5-8) following the area of the measured impedance Z of the biological object 100 with a wider pickup area of voltage electrodes (7,8) than the scattering range of the excitation current i from current electrodes (5, 6), minimizes the leakage of the excitation current outside the area of the measured impedance Z. Minimizing poorly defined current leakages up to their elimination ensures the best measurement accuracy.
10. The separation of the voltage VΔZ corresponding to the variation ΔZ of the impedance Z (Fig 8, Fig 9) and a separate demodulation of it (Fig 10) provides high accuracy measurement for the demodulated voltage DΔZ and for the determination of the corresponding impedance variations ΔZ in the measuring device (MS) 3 (Fig 1).

The method for measuring electrical bioimpedance.

For measuring the electrical bioimpedance, an active electrode system 1 corresponding to the configuration of the biological object 100 is selected and placed on the body grasping the contours of the biological object 100 in parallel with it, whereby the excitation current electrodes 5, 6 grasp the contours externally with respect to the voltage pickup electrodes 7, 8, where the position of the voltage pickup electrodes 7, 8 determines the contours on the biological object 100 to be measured (Figs 2 to 4 and Figs 11 to 17). In the case of absence of the electrode system corresponding to the required configuration, the electrodes created using a blank electrode system from strip electrodes so that the necessary configuration formed from the strips (Figs 11 to 16). An effective example would be the use of a matrix of point electrodes, in which the strip electrode paths created (Fig 17) using, for example, mechanical processing or programmable electrical connecting.

Preferred is the formation of strip electrodes through the electronic programming of the electrode matrix, whereby there are two ways to create the strip electrode paths: a) from the ready-made connections of the point electrodes interrupting (burning out) the excessive ones; b) connecting only these point electrodes that are required for creating the strip electrodes by closing the needed connections by the aid of electrical pulses. The connections and interruptions created similarly to the programming of matrix memories.

After formation of the electrodes, placing the excitation current electrodes 5, 6 and the voltage collection electrodes 7, 8 on the biological object 100 following the required contours takes place, and connecting the electrodes 5, 6 and 7, 8 to the inverting input and output of the operational amplifier 9 located in the active electrode system 1 will be done. The placing of electrodes also involves connecting the active electrode system 1 to the analog interface 2 and the generator (G) 4.

The generator (G) 4 is set to the required frequency f and the voltage VG of it is regulated to a suitable level for generating the excitation current *i* = VG/R using the current-impeding circuit 10 (Fig 2, Fig 3 and Figs 5 to 7) and directing the excitation current *i* via the excitation current electrodes 5, 6 through the electrical bioimpedance Z of the biological object 100, which is switched into the negative feedback circuit of the operational amplifier 9 between the excitation current electrodes 5, 6 (Fig 2, Fig 3). When setting the excitation current, the established restrictions on the strength of the electric current in living biological subjects (Medical Devices IEC 60601-1 - Electrical Safety EN 60601-1) must considered.

The configuration of the current-impeding circuit (R) 10 (Figs 5 to 7) and the values of the components 21-25 included therein (resistors r3, rp1 and rp2 and capacitances C1 and C2, are chosen so that the total electrical resistance R of the current-impeding circuit 10 and its dependence on the operating frequency f, would be as similar as possible to the impedance Z of the biological object being measured at the frequency Z(f).

The voltage VZ = *i*·Z created by the excitation current *i* flowing through the electrical bioimpedance Z of the biological object 100 recorded in the form of the difference between the voltages V1 and V2 on the voltage pickup electrodes 7, 8 (Fig 8, Fig 9). The voltages V1 and V2 processed in the analog interface 2 together with the output voltage VG of the generator 4 to obtain the voltage modulation VΔZ = [(V2-V1)-VG] corresponding to the change ΔZ in the impedance Z. Then the voltages VZ and VΔZ (Fig 10) are demodulated into the form DZ and DΔZ and measured in the measuring device 3, which provides an output corresponding to the impedance Z and its informative variation ΔZ, which are the results of the impedance measurement of the biological object 100 (Figs 1 to 4).

To check the correctness of measurements, the voltage V3 of the excitation current electrode 6 located at the output of the operational amplifier 9 is transferred to the measuring device 3 for evaluation, where the measurement result is compared with a predetermined voltage, exceeding of which indicates overloading of the device. Consequently, the measurement results are not correct and must cancelled. The final purpose is to measure the voltage V1 of the voltage pickup electrode 7 in the measuring device 3 (Figs 1 to 3) to verify the correctness of the contact between the excitation current electrode 5 and the object 100. The voltage V1 must be smaller than the set reference voltage. If any irregularities occur, the measuring device 3 issues an alarm signal. For the purposes of technical diagnostics, the values of the contact resistances r1 and r2 can be calculated based on the measurement results (Figs 2 to 4), and the stability and quality of the contacts between the electrodes and the body can be assessed, as well as conclusions can be drawn about the reliability of the measurements.

The advantages of the invention summed up in terms of the method.
1. The use of a strip form for designing the optimal configurations of both the excitation current electrodes 5, 6 and the voltage pickup electrodes 7, 8 according to the measurement task and the shape of the biological object 100 ensures the recording of signals containing maximum amount of information acquired from the voltage pickup electrodes 7, 8.
2. Marking the biological object 100 on the body in accordance with the configuration and position of the voltage pickup electrodes 7, 8 allows us to precisely define the area of the body impedance Z we want to measure. This ensures the true value of measurement results.
3. The use of warning signals developed based on the measurement of the electrode voltages V1 and V3 allows us to remove the measurement results corrupted by possible technical irregularities and disturbances. We achieve an increase in the reliability of medical diagnostics performed based on the bioimpedance measurement results.
4. Determination of the strength and frequency dependence of the excitation current *i* by selecting the circuit solution of the current-impeding circuit 10 (Figs 5 to 7) and setting the values of its components 21-24, the excitation current level maintained at the maximally allowed at all the excitation frequencies f. As a result, we achieve the best signal-to-noise ratio and measurement accuracy without exceeding the permitted electric current limits in biological subjects.

## Claims

1. A device for measuring electrical bioimpedance comprising a measuring device (3), a generator (4), excitation current electrodes (5, 6) and voltage pickup electrodes (7, 8), electrical contact resistances (r1, r2) between the excitation current electrodes (5, 6) and the biological object (100), **characterized in that** the device further comprises:
- an active electrode system (1), comprising an operational amplifier (9);
- a current-impeding circuit (10);
- an analog interface (2), comprising:
∘ a summator (11), having three units: a first summing unit (13), a second summing unit (14), and an amplifier (15);
∘ a demodulator (12), comprising two multipliers (16, 17), a formator (18), a low-pass filter (19), a band-pass filter (20);
∘ the voltage outputs leading to the inputs of the measuring device (3), wherein one of the inputs is connected to the excitation current electrode (6) of the operational amplifier (9) output in the active electrode system (1) for measuring the voltage V3 and the other voltage input is connected to the voltage pickup electrode (7) for measuring the voltage V1, the two subsequent inputs of the measuring device (3) are connected with two other outputs of the analog interface (2) for measuring the voltages DZ and DΔZ detected in the demodulator (12) in the analog interface (2).

2. The device according to claim 1, **characterized in that** the output of the generator (4) with voltage VG is connected to the input of the active electrode system (1) and the input of the analog interface (2), the informative voltage outputs with voltages V1 and V2 from the active electrode system (1) are connected to the inputs of the analog interface (2), and the output voltages V3 and V1 of the active electrode system (1) are directed for evaluation to the inputs of the measuring device (3).

3. The device according to claim 1, **characterized in that** the inverting input (-) of the operational amplifier (9) is connected to the one excitation current electrode (5) and the non-inverting input (+) is connected to a common ground holding the 0-potential (V=0), whereas the output of the operational amplifier (9) is connected to the second excitation current electrode (6).

4. The device according to claim 1, **characterized in that** the current-impeding circuit (10) with a resistance R is connected between the zero-potential (V≈0) inverting input (-) of the operational amplifier (9) and the output of the generator (4) with voltage VG, and the current-impeding circuit (10) comprises a frequency-dependent resistance consisting of a fixed resistor (r3) and one or several parallel resistors (rp1, rp2) connected in parallel to it through one (C1) or several (C1, C2) capacitances.

5. The device according to claim 1, **characterized in that** the non-inverting input (+) and the inverting input (-) of the summator (11) are connected to the voltage pickup electrodes (7, 8) with voltages (V1, V2) and to the subtracting input (- -) of the summator (11), which is connected to the output of the generator (4) with voltage VG.

6. The device according to claim 1, **characterized in that** the two signal inputs of the demodulator (12) are connected with the outputs of the summator (11) with voltages VZ and VΔZ, and the reference input is connected to the output of the generator (4) with voltage VG, the outputs of the demodulated voltages DZ and DΔZ are connected to inputs of the measuring device (3) that elaborates the numerical values for the impedance Z and its variation ΔZ.

7. The device according to claim 1 and 5, **characterized in that** the summator (11) comprises a first summing unit S1 (13) and a second summing unit S2 (14), whereas from the input voltage V2 at the non-inverting input (+) of the first summing unit (13) is subtracted the input voltage V1 at the inverting input (-), as a result of the subtracting, the output of the first summing unit (13) has the modulated voltage, corresponding to the impedance Z, VZ = V2 - V1, which is directed to the non-inverting input (+) of the second summing unit (14), from which, in turn, the voltage VG entering the inverting input (- -) of the second summing unit (14) from the generator (4) is subtracted, wherein the amplifier (15) connected to the output of the second summing unit (14) gives out an amplified voltage VΔZ carrying information on the impedance variation ΔZ.

8. The device according to claim 1 and 3, **characterized in that** the demodulator (12) comprises one multiplier X1 (16) and another multiplier X2 (17), a formator (18), a low-pass filter LPF (19) and a bandpass filter BPF (20), wherein the signal input of the multiplier (16) is connected to the output of the summator (11) with the voltage signal VZ and the signal input of the multiplier (17) is connected to the output of the summator (11) with the voltage signal VΔZ, the output of the formator F (18) is connected to the reference inputs of both the multipliers (16 and 17), the input of the formator F (18) is connected to the output of the generator (4) with the voltage VG, wherein the output of the one multiplier (16) is connected to the input of the low-pass filter LPF (19), the output of which has a demodulated signal DZ corresponding to the impedance Z, and the output of the other multiplier (17) is connected to the input of the bandpass filter BPF (20), the demodulated output signal DΔZ of which corresponds to the impedance variation ΔZ.

9. The device according to claim 1, **characterized in that** the generator (4) is a voltage generator.

10. The device according to claim 1, **characterized in that** the generator (4) is a current generator.

11. The device according to claim 1, **characterized in that** the excitation current electrodes and the voltage pickup electrodes are long strip electrodes placed on the biological object to follow the shape of the region, so that on one side of the region there is one excitation current electrode and one voltage pickup electrode forming a pair of electrodes, and on the other side of the region there is another voltage pickup electrode and another excitation current electrode forming another pair of electrodes.

12. The device according to claim 11, **characterized in that** at least one voltage pickup electrode is longer than one of the excitation current electrodes.

13. The device according to claims 11 and 12, **characterized in that** the strip electrodes are designed to follow linearly the region to be measured of biological object.

14. The device according to claim 11 and 12, **characterized in that** the strip electrodes are formed to follow the region of biological object in an arched shape.

15. The device according to claim 1 and 14, **characterized in that** at least one of the strip electrodes connected at the ends of the strip to form a closed contour to surround the circular round-shape regions.

16. The device according to claim 1 and 15, **characterized in that** the innermost excitation current electrode is designed to a surface electrode.

17. The device according to claim 1 and 15, **characterized in that** the innermost excitation current electrode is designed to a point electrode.

18. The device according to claim 1 and 15, **characterized in that** the innermost excitation current electrode is designed to a line electrode.

19. The device according to claim 1 and 15, **characterized in that** one excitation current electrode and one voltage pickup electrode are joined together into a single common electrode resulting a three-electrode electrode system.

20. The device according to claim 11, 12 and 13, **characterized in that** one or more strip electrodes are formed from the point and line electrodes, wherein point electrodes are in electric interconnections using programmable connecting.

21. A method for continuous monitoring of electrical bioimpedance measurement according to claims 1 to 20 of the device, comprising the steps of:
- placing the electrodes of the excitation current and the electrodes of the voltage pickup on the biological object;
- generating an electrical excitation current;
- directing the excitation current through the excitation current electrodes of the biological object;
- modulating the voltage generated by the excitation current in the biological object by the electrical bioimpedance Z of the region of object;
- recording the voltage modulated by the electrical bioimpedance Z from the voltage pickup electrodes;
- measuring the value of the voltage from the voltage pickups and deriving the value of the electrical bioimpedance Z by dividing the voltage drop measured from the voltage pickup electrodes by the value of the excitation current directed through the biological object,
**characterized in that** the method additionally comprises the following steps:
- generating the electrical excitation voltage with respect to the common ground potential (V = 0) of the device;
- transforming of the generated electrical excitation voltage into the excitation current passing through the biological object by means of the current-impeding circuit;
- directing the excitation current to the inverting input of a negative feedback operational amplifier;
- incorporating the biological object to be measured into the negative serial feedback loop between the two excitation current electrodes connecting the output of the operational amplifier to its inverting input in the active electrode system;
- obtaining the electrical voltages V1 and V2 from the two voltage pickup electrodes located on the biological object;
- demodulating the difference of voltages VZ = (V2-V1) obtained from the voltage pickup electrodes and directing the demodulation voltage DZ to the measuring device (3) in order to gain measured and numerically presented results of the size of the electrical bioimpedance Z;
- extraction of the voltage of variation part ΔZ of the electrical bioimpedance Z from the difference of the voltages (V2-V1) by subtraction and amplification of generator voltage (VG): VΔZ = (V2-V1)-VG;
- demodulating the voltage VΔZ of variation part ΔZ of electrical bioimpedance Z and directing the demodulated voltage DΔZ to the measuring device to obtain the numerically presented measurement results of the variation part ΔZ of the impedance Z;
- taking the electrical voltage V3 from the output of the operational amplifier located in the active electrode system and directing it to the measuring device (3) for measurement and evaluation;
- taking the electrical voltage V1 from the voltage pickup electrode and directing it to the measuring device for measurement and evaluation;
- designing and installing the excitation current electrodes and the voltage pickup electrodes.

22. The method according to claim 21, **characterized in that** the steps of forming and installing the electrodes are:
- forming the electrodes from long and narrow electrically conductive materials with a strip-shaped design;
- placing the strip-shaped excitation current and voltage pickup electrodes on the body surface surrounding the selected regions of the biological object so that, following the surface shape of the object, one excitation current electrode (5) and one voltage pickup electrode (7) which form the first electrode pair (5, 7) are placed on one side of the object as well as the second electrode pair (6, 8) formed from the second excitation current electrode (6) and the second voltage pickup electrode (8) are placed on the other side of the object:
- forming the arc-shaped electrodes surrounding the selected region of biological objects;
- forming circular electrodes surrounding the selected region of biological objects;
- forming the electrodes as the closed concentric circles;
- forming one of the electrodes as an internal surface electrode;
- forming one of the electrodes as a point electrode;
- forming the electrodes as a series of point electrodes connected with each other;
- designing the single strip-shaped voltage pickup electrode as common electrode comprising one excitation current electrode and one voltage pickup electrode as a pair, wherein the excitation current electrode involved in the pair is for the purpose for the collectable voltage to grip the whole voltage drop generated by the excitation current.

## Patentansprüche

1. Eine Vorrichtung zum Messen elektrischer Bioimpedanz, beinhaltend eine Messvorrichtung (3), einen Generator (4), Erregerstromelektroden (5, 6) und Spannungsauffängerelektroden (7, 8), elektrische Kontaktwiderstände (r1, r2) zwischen den Erregerstromelektroden (5, 6) und dem biologischen Objekt (100), **dadurch gekennzeichnet, dass** die Vorrichtung ferner Folgendes beinhaltet:
- ein aktives Elektrodensystem (1), beinhaltend einen Operationsverstärker (9);
- eine stromhemmende Schaltung (10);
- eine analoge Schnittstelle (2), beinhaltend:
∘ einen Summierer (11), der drei Einheiten aufweist: eine erste Summiereinheit (13), eine zweite Summiereinheit (14) und einen Verstärker (15);
∘ einen Demodulator (12), beinhaltend zwei Multiplizierer (16, 17), einen Formator (18), ein Tiefpassfilter (19), ein Bandpassfilter (20);
∘ die Spannungsausgänge, die zu den Eingängen der Messvorrichtung (3) führen, wobei einer der Eingänge zum Messen der Spannung V3 mit der Erregerstromelektrode (6) des Ausgangs des Operationsverstärkers (9) in dem aktiven Elektrodensystem (1) verbunden ist und der andere Spannungseingang zum Messen der Spannung V1 mit der Spannungsauffängerelektrode (7) verbunden ist, wobei die zwei nachfolgenden Eingänge der Messvorrichtung (3) zum Messen der Spannungen DZ und DΔZ, die in dem Demodulator (12) in der analogen Schnittstelle (2) detektiert werden, mit zwei anderen Ausgängen der analogen Schnittstelle (2) verbunden sind.

2. Vorrichtung gemäß Anspruch 1, **dadurch gekennzeichnet, dass** der Ausgang des Generators (4) mit der Spannung VG mit dem Eingang des aktiven Elektrodensystems (1) und dem Eingang der analogen Schnittstelle (2) verbunden ist, die informativen Spannungsausgänge mit den Spannungen V1 und V2 von dem aktiven Elektrodensystem (1) mit den Eingängen der analogen Schnittstelle (2) verbunden sind und die Ausgangsspannungen V3 und V1 des aktiven Elektrodensystems (1) zur Auswertung zu den Eingängen der Messvorrichtung (3) geleitet werden.

3. Vorrichtung gemäß Anspruch 1, **dadurch gekennzeichnet, dass** der invertierende Eingang (-) des Operationsverstärkers (9) mit der einen Erregerstromelektrode (5) verbunden ist und der nichtinvertierende Eingang (+) mit einer gemeinsamen Masse verbunden ist, die das 0-Potential (V = 0) hält, wohingegen der Ausgang des Operationsverstärkers (9) mit der zweiten Erregerstromelektrode (6) verbunden ist.

4. Vorrichtung gemäß Anspruch 1, **dadurch gekennzeichnet, dass** die stromhemmende Schaltung (10) mit einem Widerstand R zwischen dem invertierenden Nullpotential(V ≈ 0)-Eingang (-) des Operationsverstärkers (9) und dem Ausgang des Generators (4) mit der Spannung VG verbunden ist und die stromhemmende Schaltung (10) einen frequenzabhängigen Widerstand beinhaltet, der aus einem festen Widerstandselement (r3) und einem oder mehreren parallelen Widerstandselementen (rp1, rp2), die durch eine (C1) oder mehrere (C1, C2) Kapazitäten parallel dazu geschaltet sind, besteht.

5. Vorrichtung gemäß Anspruch 1, **dadurch gekennzeichnet, dass** der nichtinvertierende Eingang (+) und der invertierende Eingang (-) des Summierers (11) mit den Spannungsauffängerelektroden (7, 8) mit den Spannungen (V1, V2) und mit dem subtrahierenden Eingang (- -) des Summierers (11), der mit dem Ausgang des Generators (4) mit Spannung VG verbunden ist, verbunden sind.

6. Vorrichtung gemäß Anspruch 1, **dadurch gekennzeichnet, dass** die zwei Signaleingänge des Demodulators (12) mit den Ausgängen des Summierers (11) mit den Spannungen VZ und VΔZ verbunden sind und der Referenzeingang mit dem Ausgang des Generators (4) mit der Spannung VG verbunden ist, wobei die Ausgänge der demodulierten Spannungen DZ und DΔZ mit Eingängen der Messvorrichtung (3) verbunden sind, die die numerischen Werte für die Impedanz Z und deren Variation ΔZ ausarbeitet.

7. Vorrichtung gemäß Anspruch 1 und 5, **dadurch gekennzeichnet, dass** der Summierer (11) eine erste Summiereinheit S1 (13) und eine zweite Summiereinheit S2 (14) beinhaltet, wohingegen die Eingangsspannung V1 an dem invertierenden Eingang (-) von der Eingangsspannung V2 an dem nichtinvertierenden Eingang (+) der ersten Summiereinheit (13) subtrahiert wird, wobei als Ergebnis der Subtraktion der Ausgang der ersten Summiereinheit (13) die modulierte Spannung, entsprechend der Impedanz Z, VZ = V2 - V1 aufweist, die zu dem nichtinvertierenden Eingang (+) der zweiten Summiereinheit (14) geleitet wird und von der wiederum die Spannung VG, die von dem Generator (4) in den invertierenden Eingang (- -) der zweiten Summiereinheit (14) eintritt, subtrahiert wird, wobei der Verstärker (15), der mit dem Ausgang der zweiten Summiereinheit (14) verbunden ist, eine verstärkte Spannung VΔZ ausgibt, die Informationen über die Impedanzvariation ΔZ trägt.

8. Vorrichtung gemäß Anspruch 1 und 3, **dadurch gekennzeichnet, dass** der Demodulator (12) einen Multiplizierer X1 (16) und einen anderen Multiplizierer X2 (17), einen Formator (18), ein Tiefpassfilter LPF (19) und ein Bandpassfilter BPF (20) beinhaltet, wobei der Signaleingang des Multiplizierers (16) mit dem Ausgang des Summierers (11) mit dem Spannungssignal VZ verbunden ist und der Signaleingang des Multiplizierers (17) mit dem Ausgang des Summierers (11) mit dem Spannungssignal VΔZ verbunden ist, der Ausgang des Formators F (18) mit den Referenzeingängen der beiden Multiplizierer (16 und 17) verbunden ist, der Eingang des Formators F (18) mit dem Ausgang des Generators (4) mit der Spannung VG verbunden ist, wobei der Ausgang des einen Multiplizierers (16) mit dem Eingang des Tiefpassfilters LPF (19) verbunden ist, dessen Ausgang ein demoduliertes Signal DZ aufweist, das der Impedanz Z entspricht, und der Ausgang des anderen Multiplizierers (17) mit dem Eingang des Bandpassfilters BPF (20) verbunden ist, dessen demoduliertes Ausgangssignal DΔZ der Impedanzvariation ΔZ entspricht.

9. Vorrichtung gemäß Anspruch 1, **dadurch gekennzeichnet, dass** der Generator (4) ein Spannungsgenerator ist.

10. Vorrichtung gemäß Anspruch 1, **dadurch gekennzeichnet, dass** der Generator (4) ein Stromgenerator ist.

11. Vorrichtung gemäß Anspruch 1, **dadurch gekennzeichnet, dass** die Erregerstromelektroden und die Spannungsauffängerelektroden lange Streifenelektroden sind, die auf dem biologischen Objekt platziert sind, um der Form des Bereichs zu folgen, sodass sich auf einer Seite des Bereichs eine Erregerstromelektrode und eine Spannungsauffängerelektrode befinden, die ein Elektrodenpaar bilden, und sich auf der anderen Seite des Bereichs eine andere Spannungsauffängerelektrode und eine andere Erregerstromelektrode befinden, die ein anderes Elektrodenpaar bilden.

12. Vorrichtung gemäß Anspruch 11, **dadurch gekennzeichnet, dass** mindestens eine Spannungsauffängerelektrode länger als eine der Erregerstromelektroden ist.

13. Vorrichtung gemäß den Ansprüchen 11 und 12, **dadurch gekennzeichnet, dass** die Streifenelektroden gestaltet sind, dem zu messenden Bereich des biologischen Objekts linear zu folgen.

14. Vorrichtung gemäß Anspruch 11 und 12, **dadurch gekennzeichnet, dass** die Streifenelektroden gebildet sind, dem Bereich des biologischen Objekts in einer Bogenform zu folgen.

15. Vorrichtung gemäß Anspruch 1 und 14, **dadurch gekennzeichnet, dass** mindestens eine der Streifenelektroden an den Enden des Streifens verbunden ist, um eine geschlossene Kontur zu bilden, um die kreisförmigen Rundform-Bereiche zu umgeben.

16. Vorrichtung gemäß Anspruch 1 und 15, **dadurch gekennzeichnet, dass** die innerste Erregerstromelektrode als eine Oberflächenelektrode gestaltet ist.

17. Vorrichtung gemäß Anspruch 1 und 15, **dadurch gekennzeichnet, dass** die innerste Erregerstromelektrode als eine Punktelektrode gestaltet ist.

18. Vorrichtung gemäß Anspruch 1 und 15, **dadurch gekennzeichnet, dass** die innerste Erregerstromelektrode als eine Linienelektrode gestaltet ist.

19. Vorrichtung gemäß Anspruch 1 und 15, **dadurch gekennzeichnet, dass** eine Erregerstromelektrode und eine Spannungsauffängerelektrode zu einer einzigen gemeinsamen Elektrode zusammengefügt sind, was ein Dreielektroden-Elektrodensystem ergibt.

20. Vorrichtung gemäß Anspruch 11, 12 und 13, **dadurch gekennzeichnet, dass** aus den Punkt- und Linienelektroden eine oder mehrere Streifenelektroden gebildet sind, wobei die Punktelektroden unter Verwendung einer programmierbaren Verbindung in elektrischen Zusammenschaltungen vorliegen.

21. Ein Verfahren zum kontinuierlichen Überwachen der Messung elektrischer Bioimpedanz gemäß den Ansprüchen 1 bis 20 der Vorrichtung, beinhaltend die folgenden Schritte:
- Platzieren der Elektroden des Erregerstroms und der Elektroden des Spannungsauffängers auf dem biologischen Objekt;
- Erzeugen eines elektrischen Erregerstroms;
- Leiten des Erregerstroms durch die Erregerstromelektroden des biologischen Objekts;
- Modulieren der Spannung, die durch den Erregerstrom in dem biologischen Objekt erzeugt wird, durch die elektrische Bioimpedanz Z des Bereichs des Objekts;
- Aufzeichnen der Spannung, die durch die elektrische Bioimpedanz Z moduliert wird, von den Spannungsauffängerelektroden;
- Messen des Werts der Spannung von den Spannungsauffängern und Ableiten des Werts der elektrischen Bioimpedanz Z durch Dividieren des Spannungsabfalls, der von den Spannungsauffängerelektroden gemessen wird, durch den Wert des Erregerstroms, der durch das biologische Objekt geleitet wird,
**dadurch gekennzeichnet, dass** das Verfahren zusätzlich die folgenden Schritte beinhaltet:
- Erzeugen der elektrischen Erregerspannung in Bezug auf das gemeinsame Massepotential (V = 0) der Vorrichtung;
- Transformieren der erzeugten elektrischen Erregerspannung in den Erregerstrom, der durch das biologische Objekt fließt, mittels der stromhemmenden Schaltung;
- Leiten des Erregerstroms zu dem invertierenden Eingang eines Operationsverstärkers mit negativer Rückkopplung;
- Einbinden des zu messenden biologischen Objekts in die negative serielle Rückkopplungsschleife zwischen den zwei Erregerstromelektroden, die den Ausgang des Operationsverstärkers mit seinem invertierenden Eingang in dem aktiven Elektrodensystem verbindet;
- Erhalten der elektrischen Spannungen V1 und V2 von den zwei Spannungsauffängerelektroden, die sich auf dem biologischen Objekt befinden;
- Demodulieren der Differenz der Spannungen VZ = (V2 - V1), die von den Spannungsauffängerelektroden erhalten werden, und Leiten der Demodulationsspannung DZ zu der Messvorrichtung (3), um gemessene und numerisch präsentierte Ergebnisse der Größe der elektrischen Bioimpedanz Z zu erlangen;
- Extrahieren der Spannung des Variationsteils ΔZ der elektrischen Bioimpedanz Z aus der Differenz der Spannungen (V2 - V1) durch Subtraktion und Verstärkung der Generatorspannung (VG): VΔZ = (V2 - V1) - VG;
- Demodulieren der Spannung VΔZ des Variationsteils ΔZ der elektrischen Bioimpedanz Z und Leiten der demodulierten Spannung DΔZ zu der Messvorrichtung, um die numerisch präsentierten Messergebnisse des Variationsteils ΔZ der Impedanz Z zu erhalten;
- Abgreifen der elektrischen Spannung V3 von dem Ausgang des Operationsverstärkers, der sich in dem aktiven Elektrodensystem befindet, und Leiten derselben zu der Messvorrichtung (3) zur Messung und Auswertung;
- Abgreifen der elektrischen Spannung V1 von der Spannungsauffängerelektrode und Leiten derselben zu der Messvorrichtung zur Messung und Auswertung;
- Gestalten und Installieren der Erregerstromelektroden und der Spannungsauffängerelektroden.

22. Verfahren gemäß Anspruch 21, **dadurch gekennzeichnet, dass** die Schritte des Bildens und Installierens der Elektroden die Folgenden sind:
- Bilden der Elektroden aus langen und schmalen elektrisch leitfähigen Materialien mit einer streifenförmigen Gestaltung;
- Platzieren der streifenförmigen Erregerstrom- und Spannungsauffängerelektroden auf der Körperoberfläche, die die ausgewählten Bereiche des biologischen Objekts umgibt, sodass, der Oberflächenform des Objekts folgend, eine Erregerstromelektrode (5) und eine Spannungsauffängerelektrode (7), die das erste Elektrodenpaar (5, 7) bilden, auf einer Seite des Objekts platziert werden sowie das zweite Elektrodenpaar (6, 8), das aus der zweiten Erregerstromelektrode (6) und der zweiten Spannungsauffängerelektrode (8) gebildet ist, auf der anderen Seite des Objekts platziert wird;
- Bilden der bogenförmigen Elektroden, die den ausgewählten Bereich von biologischen Objekten umgeben;
- Bilden kreisförmiger Elektroden, die den ausgewählten Bereich von biologischen Objekten umgeben;
- Bilden der Elektroden als die geschlossenen konzentrischen Kreise;
- Bilden einer der Elektroden als eine innere Oberflächenelektrode;
- Bilden einer der Elektroden als eine Punktelektrode;
- Bilden der Elektroden als eine Reihe von Punktelektroden, die miteinander verbunden sind;
- Gestalten der einzelnen streifenförmigen Spannungsauffängerelektrode als eine gemeinsame Elektrode, die eine Erregerstromelektrode und eine Spannungsauffängerelektrode als ein Paar beinhaltet, wobei die Erregerstromelektrode, die an dem Paar beteiligt ist, dem Zweck dient, dass die sammelbare Spannung den gesamten durch den Erregerstrom erzeugten Spannungsabfall erfasst.

## Revendications

1. Un dispositif pour mesurer une bioimpédance électrique comprenant un dispositif de mesure (3), un générateur (4), des électrodes de courant d'excitation (5, 6) et des électrodes de captage de tension (7, 8), des résistances contacts électriques (r1, r2) entre les électrodes de courant d'excitation (5, 6) et l'objet biologique (100), **caractérisé en ce que** le dispositif comprend en outre :
- un système d'électrodes actives (1), comprenant un amplificateur opérationnel (9) ;
- un circuit d'impédance au courant (10) ;
- une interface analogique (2), comprenant :
∘ un sommateur (11), ayant trois unités : une première unité de sommation (13), une deuxième unité de sommation (14), et un amplificateur (15) ;
∘ un démodulateur (12), comprenant deux multiplicateurs (16, 17), un formateur (18), un filtre passe-bas (19), un filtre passe-bande (20) ;
∘ les sorties de tension conduisant aux entrées du dispositif de mesure (3), où une des entrées est connectée à l'électrode de courant d'excitation (6) de la sortie d'amplificateur opérationnel (9) dans le système d'électrodes actives (1) pour mesurer la tension V3 et l'autre entrée de tension est connectée à l'électrode de captage de tension (7) pour mesurer la tension V1, les deux entrées subséquentes du dispositif de mesure (3) étant connectées à deux autres sorties de l'interface analogique (2) pour mesurer les tensions DZ et DΔZ détectées dans le démodulateur (12) dans l'interface analogique (2).

2. Le dispositif selon la revendication 1, **caractérisé en ce que** la sortie du générateur (4) avec une tension VG est connectée à l'entrée du système d'électrodes actives (1) et à l'entrée de l'interface analogique (2), les sorties de tension informatives avec des tensions V1 et V2 provenant du système d'électrodes actives (1) sont connectées aux entrées de l'interface analogique (2), et les tensions de sortie V3 et V1 du système d'électrodes actives (1) sont dirigées pour évaluation vers les entrées du dispositif de mesure (3).

3. Le dispositif selon la revendication 1, **caractérisé en ce que** l'entrée inverseuse (-) de l'amplificateur opérationnel (9) est connectée à cette une électrode de courant d'excitation (5) et l'entrée non inverseuse (+) est connectée à une masse commune maintenant le potentiel 0 (V = 0), tandis que la sortie de l'amplificateur opérationnel (9) est connectée à la deuxième électrode de courant d'excitation (6).

4. Le dispositif selon la revendication 1, **caractérisé en ce que** le circuit d'impédance au courant (10) avec une résistance R est connecté entre l'entrée inverseuse (-) de potentiel zéro (V ≈ 0) de l'amplificateur opérationnel (9) et la sortie du générateur (4) à tension VG, et le circuit d'impédance au courant (10) comprend une résistance dépendant de la fréquence consistant en une résistance fixe (r3) et une ou plusieurs résistances parallèles (rp1, rp2) connectées en parallèle à celle-ci par l'intermédiaire d'un (C1) ou de plusieurs (C1, C2) condensateurs.

5. Le dispositif selon la revendication 1, **caractérisé en ce que** l'entrée non inverseuse (+) et l'entrée inverseuse (-) du sommateur (11) sont connectées aux électrodes de captage de tension (7, 8) avec des tensions (V1, V2) et à l'entrée de soustraction (- -) du sommateur (11), qui est connectée à la sortie du générateur (4) à tension VG.

6. Le dispositif selon la revendication 1, **caractérisé en ce que** les deux entrées de signal du démodulateur (12) sont connectées aux sorties du sommateur (11) avec des tensions VZ et VΔZ, et l'entrée de référence est connectée à la sortie du générateur (4) avec la tension VG, les sorties des tensions démodulées DZ et DΔZ étant connectées à des entrées du dispositif de mesure (3) qui élabore les valeurs numériques pour l'impédance Z et sa variation ΔZ.

7. Le dispositif selon les revendications 1 et 5, **caractérisé en ce que** le sommateur (11) comprend une première unité de sommation S1 (13) et une deuxième unité de sommation S2 (14), tandis que de la tension d'entrée V2 à l'entrée non inverseuse (+) de la première unité de sommation (13) est soustraite la tension d'entrée V1 à l'entrée inverseuse (-), en conséquence de la soustraction, la sortie de la première unité de sommation (13) a la tension modulée, correspondant à l'impédance Z, VZ = V2 - V1, qui est dirigée vers l'entrée non inverseuse (+) de la deuxième unité de sommation (14), de laquelle, à son tour, la tension VG pénétrant dans l'entrée inverseuse (- -) de la deuxième unité de sommation (14) depuis le générateur (4) est soustraite, où l'amplificateur (15) connecté à la sortie de la deuxième unité de sommation (14) délivre une tension amplifiée VΔZ portant des informations sur la variation d'impédance ΔZ.

8. Le dispositif selon les revendications 1 et 3, **caractérisé en ce que** le démodulateur (12) comprend un multiplicateur X1 (16) et un autre multiplicateur X2 (17), un formateur (18), un filtre passe-bas LPF (19) et un filtre passe-bande BPF (20), où l'entrée de signal du multiplicateur (16) est connectée à la sortie du sommateur (11) avec le signal de tension VZ et l'entrée de signal du multiplicateur (17) est connectée à la sortie du sommateur (11) avec le signal de tension VΔZ, la sortie du formateur F (18) est connectée aux entrées de référence des deux multiplicateurs (16 et 17), l'entrée du formateur F (18) est connectée à la sortie du générateur (4) avec la tension VG, où la sortie de cet un multiplicateur (16) est connectée à l'entrée du filtre passe-bas LPF (19), dont la sortie a un signal démodulé DZ correspondant à l'impédance Z, et la sortie de l'autre multiplicateur (17) est connectée à l'entrée du filtre passe-bande BPF (20), dont le signal de sortie démodulé DΔZ correspond à la variation d'impédance ΔZ.

9. Le dispositif selon la revendication 1, **caractérisé en ce que** le générateur (4) est un générateur de tension.

10. Le dispositif selon la revendication 1, **caractérisé en ce que** le générateur (4) est un générateur de courant.

11. Le dispositif selon la revendication 1, **caractérisé en ce que** les électrodes de courant d'excitation et les électrodes de captage de tension sont des électrodes en bandes longues placées sur l'objet biologique pour suivre la forme de la région, de sorte que sur un côté de la région il y a une électrode de courant d'excitation et une électrode de captage de tension formant une paire d'électrodes, et sur l'autre côté de la région il y a une autre électrode de captage de tension et une autre électrode de courant d'excitation formant une autre paire d'électrodes.

12. Le dispositif selon la revendication 11, **caractérisé en ce qu'**au moins une électrode de captage de tension est plus longue qu'une des électrodes de courant d'excitation.

13. Le dispositif selon les revendications 11 et 12, **caractérisé en ce que** les électrodes en bandes sont conçues pour suivre linéairement la région à mesurer de l'objet biologique.

14. Le dispositif selon les revendications 11 et 12, **caractérisé en ce que** les électrodes en bandes sont formées pour suivre la région de l'objet biologique en une forme arquée.

15. Le dispositif selon les revendications 1 et 14, **caractérisé en ce qu'**au moins une des électrodes en bandes est connectée aux extrémités de la bande pour former un contour fermé afin d'entourer les régions de forme ronde circulaires.

16. Le dispositif selon les revendications 1 et 15, **caractérisé en ce que** l'électrode de courant d'excitation la plus interne est conçue comme une électrode de surface.

17. Le dispositif selon les revendications 1 et 15, **caractérisé en ce que** l'électrode de courant d'excitation la plus interne est conçue comme une électrode de pointe.

18. Le dispositif selon les revendications 1 et 15, **caractérisé en ce que** l'électrode de courant d'excitation la plus interne est conçue comme une électrode de ligne.

19. Le dispositif selon les revendications 1 et 15, **caractérisé en ce qu'**une électrode de courant d'excitation et une électrode de captage de tension sont jointes ensemble en une électrode commune unique résultant en un système d'électrodes à trois électrodes.

20. Le dispositif selon la revendication 11, 12 et 13, **caractérisé en ce qu'**une ou plusieurs électrodes en bandes sont formées à partir des électrodes de pointe et de ligne, dans lequel les électrodes de pointe sont dans des interconnexions électriques en utilisant une connexion programmable.

21. Un procédé pour la surveillance continue d'une mesure de bioimpédance électrique selon les revendications 1 à 20 du dispositif, comprenant les étapes consistant à :
- placer les électrodes du courant d'excitation et les électrodes du captage de tension sur l'objet biologique ;
- générer un courant d'excitation électrique ;
- diriger le courant d'excitation à travers les électrodes de courant d'excitation de l'objet biologique ;
- moduler la tension générée par le courant d'excitation dans l'objet biologique par la bioimpédance électrique Z de la région de l'objet ;
- enregistrer la tension modulée par la bioimpédance électrique Z à partir des électrodes de captage de tension ;
- mesurer la valeur de la tension à partir des captages de tension et dériver la valeur de la bioimpédance électrique Z en divisant la chute de tension mesurée à partir des électrodes de captage de tension par la valeur du courant d'excitation dirigé à travers l'objet biologique,
**caractérisé en ce que** le procédé comprend de surcroît les étapes suivantes :
- générer la tension d'excitation électrique par rapport au potentiel de masse commune (V = 0) du dispositif ;
- transformer la tension d'excitation électrique générée en ce courant d'excitation passant à travers l'objet biologique au moyen du circuit d'impédance au courant ;
- diriger le courant d'excitation vers l'entrée inverseuse d'un amplificateur opérationnel à rétroaction négative ;
- incorporer l'objet biologique à mesurer dans la boucle de rétroaction en série négative entre les deux électrodes de courant d'excitation connectant la sortie de l'amplificateur opérationnel à son entrée inverseuse dans le système d'électrodes actives ;
- obtenir les tensions électriques V1 et V2 à partir des deux électrodes de captage de tension situées sur l'objet biologique ;
- démoduler la différence de tensions VZ = (V2-V1) obtenue à partir des électrodes de captage de tension et diriger la tension de démodulation DZ vers le dispositif de mesure (3) afin d'acquérir des résultats mesurés et présentés numériquement de la taille de la bioimpédance électrique Z ;
- extraire la tension de la partie de variation ΔZ de la bioimpédance électrique Z à partir de la différence des tensions (V2-V1) par soustraction et amplification de la tension de générateur (VG) : VΔZ = (V2-V1)-VG ;
- démoduler la tension VΔZ de la partie de variation ΔZ de la bioimpédance électrique Z et diriger la tension démodulée DΔZ vers le dispositif de mesure afin d'obtenir les résultats de mesure présentés numériquement de la partie de variation ΔZ de l'impédance Z ;
- prélever la tension électrique V3 à partir de la sortie de l'amplificateur opérationnel situé dans le système d'électrodes actives et la diriger vers le dispositif de mesure (3) pour mesure et évaluation ;
- prélever la tension électrique V1 à partir de l'électrode de captage de tension et la diriger vers le dispositif de mesure pour mesure et évaluation ;
- concevoir et installer les électrodes de courant d'excitation et les électrodes de captage de tension.

22. Le procédé selon la revendication 21, **caractérisé en ce que** les étapes consistant à former et installer les électrodes consistent à :
- former les électrodes à partir de matériaux électriquement conducteurs longs et étroits avec une conception en forme de bande ;
- placer les électrodes de courant d'excitation et de captage de tension en forme de bande sur la surface de corps entourant les régions sélectionnées de l'objet biologique de sorte que, suivant la forme de surface de l'objet, une électrode de courant d'excitation (5) et une électrode de captage de tension (7) qui forment la première paire d'électrodes (5, 7) sont placées sur un côté de l'objet tout comme la deuxième paire d'électrodes (6, 8) formée à partir de la deuxième électrode de courant d'excitation (6) et de la deuxième électrode de captage de tension (8) sont placées sur l'autre côté de l'objet ;
- former les électrodes en forme d'arc entourant la région sélectionnée d'objets biologiques ;
- former des électrodes circulaires entourant la région sélectionnée d'objets biologiques ;
- former les électrodes comme les cercles concentriques fermés ;
- former l'une des électrodes comme une électrode de surface interne ;
- former l'une des électrodes comme une électrode de pointe ;
- former les électrodes comme une série d'électrodes de pointe connectées les unes aux autres ;
- concevoir l'électrode de captage de tension en forme de bande unique telle une électrode commune comprenant une électrode de courant d'excitation et une électrode de captage de tension comme une paire, dans lequel l'électrode de courant d'excitation impliquée dans la paire a pour but que la tension collectable saisisse l'entièreté de la chute de tension générée par le courant d'excitation.
